# EUROPEAN PATENT APPLICATION

(11) **EP 1 577 379 A1**
(43) Date of publication of application: **21.09.2005**
(21) Application number: 05075703.8
(22) Date of filing: 26.03.2002
(51) Int. Cl.: C12N 1/20, C12N 5/00

(54) **Defined culture medium**

(30) Priority: 26.03.2001 GB 0107525
(62) Divisional of application: 02733133.9
(71) Applicant: Chiron SRL, 53100 Siena (IT)
(72) Inventor: Kapat, Arnab, 53100 Siena (IT); Olivieri, Roberto, 53100 Siena (IT)
(74) Representative: Marshall, Cameron John

(57) **Abstract**

A cell culture medium comprising glycerol or glucose, an ammonium salt, sodium polyphosphate, a magnesium salt, a potassium salt and trace elements, and having a pH between 5.5 and 8.0. The medium is particularly suitable for culturing *E*.*coli* for the expression of heterologous proteins *(e.g.* NAP from *H.pylori*).

## Description

### TECHNICAL FIELD

This invention relates to a medium for cultivating cells, the use of the medium to cultivate cells, obtaining desired components produced by the cells, and processes for obtaining the components.

### BACKGROUND ART

Culture media are used to grow cells in order to obtain desired components from them. Numerous desired components can be obtained, such as therapeutic proteins, cell wall components such as receptors, and carbohydrates.

Immunogenic components are a typically obtained from cells in this way for use in vaccine manufacture. [*e.g*. see *Bacterial Vaccines,* 1984, Ed. Rene Germanier Academic Press; *Bacterial Vaccines* in *Advances in Biotechnology Processes,* Vol. 13, 1990, Ed. A. Mizrahi, Wiley-Liss].

Typical prior art methods of cultivating cells use complex media, which comprise undefined components such as casamino acids and fetal calf serum (FCS). These components are used in the belief that they are necessary to obtain sufficient cell growth.

When pathogenic bacteria are cultured in order to obtain a desired immunogenic component, it has been considered necessary to include compounds of animal origin (blood, brain, heart infusion, meat, *etc.)* in the culture medium. For instance: *C.tetani* is grown in media containing a heart infusion and an enzymatic digest of casein; *C.diphtheriae* requires a beef infusion; *H.pylori* is grown in media containing peptamin and tryptone; and *H.influenzae* is grown in media containing proteose peptones. World Health Organisation report series numbers 800 (1990) and 814 (1991) indicate that to grow *H.influenzae, C.diphtheriae, C.tetani* and *B.pertussis,* media comprising compounds of animal origin are required.

The requirement for proteinaceous material of animal origin in the media gives rise to concern over possible contamination of the media. In particular, concern that the media may be contaminated with the bovine spongiform encephalopathy (BSE) causative agent or other infectious and harmful agents, restricts the usefulness of any components derived from such cultures, especially in therapeutic applications.

Other culture media have been used wherein yeast or soya extracts are used in culture media. The use of material of non-animal origin, such as proteins from yeast, soy beans, cotton seeds, potatoes, *etc.,* as a media constituent for the cultivation of pathogenic bacteria, completely removes the risk of animal derived contamination, such as BSE, being transmitted into humans in any subsequent therapeutic or prophylactic applications. These materials remain undefined, however, because their exact compositions are not completely known.

There are a number of difficulties associated with using such "complex" media, such as:
― the exact compositions of the nitrogenous organic compounds varies between samples (*e.g.* two samples of FCS), and this inconsistency results in substantial variation in the batch-to-batch production level of the desired protein (Zhang & Greasham, 1999).
― kinetic studies involving mass and energy balance play an important role in predictive mathematical model building of culture, which is necessary for scaling up the process, but the limited knowledge of the chemistry of the organic compounds in complex media prevents this (Bonsignore *et al*., 1989).
― residual proteinaceous materials in the final culture broth means that downstream processing (*i.e*. purification of the desired protein) is not always easy (De Vyust, 1995) and culture proteins (*e.g.* serum proteins) may remain in the final product.

One approach for overcoming some of the disadvantages is to replace the complex medium with a defined medium. These can simplify downstream processing and ease regulatory compliance.

Chemically defined media have been used for the production of biological products, including recombinant proteins on the industrial scale (Stephenne, 1990; Comberbach *et al*., 1991; Stratakalaitis *et al*., 1991; Zhang *et al*., 1998). Since the components used are usually simple chemical compounds and their amount and structures are known, defined media have been successfully employed for microbial biochemical studies where minimal interactions with the medium and reproducible results are critical and where lot-to-lot consistency is required. However, many of these media require the microorganism to synthesise all its cellular components, so fermentation performance tends to be poorer than that achieved with complex media where compounds such as amino acids and vitamins are present as constituents.

The main difficulty with defined media, therefore, is to maintain the same level of productivity as with complex media, or at least to provide a level of productivity sufficient to generate enough of a desired component for commercialisation or experimental trials.

### DISCLOSURE OF THE INVENTION

The present invention provides a cell culture medium comprising glycerol or glucose, an ammonium salt, sodium polyphosphate, a magnesium salt, a potassium salt and trace elements.

More particularly, the invention provides a cell culture medium comprising:
glucose or glycerol : 6.0 to 150.0 g/l;
ammonium salts : 2.0 to 9.0 g/l;
sodium polyphosphate : 0.6 to 12.0 g/l;
magnesium salts : 0.1 to 6.0 g/l;
potassium salts : 0.1 to 8.0 g/l; and
trace elements,
and having a pH between about 6.0 and about 8.0.

The cell culture medium is for cultivating cells. The term "cultivating" describes the maintenance of, and preferably the growth (*i.e.* increase in biomass) of, cells. Preferably the cell culture medium is used to culture cells which produce a desired component

The medium comprises glucose or glycerol as a carbon source and an ammonium salt as a nitrogen source. The carbon/nitrogen ratio of the medium will typically be between about 4 and about 20, and usually between 8 and 16. It is preferred to use a carbon/nitrogen ratio of between about 9 and about 10 e.g. about 9.66. Another preferred carbon/nitrogen ratio is between about 14 and about 15 *e.g.* about 14.4.

The carbon source is preferably present at less than 100 g/l. For example, it may be present at 6.0 to 75.0 g/l, more preferably 35 to 41 g/l, even more preferably 37 to 39 g/l, and most preferably 38±0.05 g/l. A level of 50 to 60 g/l, more preferably 55 to 57 g/l, and most preferably around 56.6 g/l is also suitable. Glycerol should be used in preference to glucose.

The nitrogen source is preferably present at 6 to 8.5 g/l, more preferably 7 to 7.5 g/l, and most preferably 7.26±0.012 g/l. Suitable ammonium salts include NH₄Cl, (NH₄)₂HPO₄, and (NH₄)₂SO₄. The preferred salt is (NH₄)₂SO₄ as chloride ions make pH control more difficult and (ortho)phosphate ions induce the formation of insoluble metallophosphates with any metal cations in the medium.

At a fixed (NH₄)₂SO₄ concentration of 7.26 g/l, a C:N ratio of 9.66 corresponds to a glycerol concentration of 38.0 g/l, and a C:N ratio of 14.4 corresponds to 56.6 g/l glycerol.

The medium comprises sodium polyphosphate as a phosphorus source. This is very soluble in water, does not form precipitates with other metal cations, and can act as an ATP substitute and a Pᵢ reservoir. (NaPO₃)₆ and (NaPO₃)₇ are essentially interchangeable and can be used in combination or individually, but (NaPO₃)₇ is preferred. The medium preferably comprises 3 to 8 g/l sodium polyphosphate, more preferably 3.5 to 4.5 g/l sodium polyphosphate, and most preferably about 4±0.04 g/l sodium polyphosphate. Another preferred range is 6.5 to 7.5 g/l, more preferably about 7.1±0.07 g/l.

The medium comprises a magnesium salt as a source of magnesium. Any soluble magnesium salt can be used, but MgSO₄.7H₂O is preferred. The medium preferably comprises 1 to 4 g/l magnesium salt, more preferably 1.50 to 1.75 g/l magnesium salt, and most preferably 1.63±0.015 g/l magnesium salt. Another preferred range is 2 to 3 g/l, more preferably about 2.94±0.03 g/l.

The medium comprises a potassium salt as a source of potassium. Any soluble potassium salt can be used *(e.g.* KCl, KNO₃, K₃PO₄ *etc.)* but K₂SO₄ is preferred. The medium preferably comprises 6 to 8 g/l potassium salt, more preferably 6.0 to 7.5 g/l potassium salt, and most preferably 7.0±0.01 g/l potassium salt.

The trace elements in the medium will typically comprise salts of microelements such as Fe, Zn, Co, Mo, Cu, Mn, B and Se. These may be added to the medium in the form of a trace element solution, at a concentration of 0.8 to 1.2 ml/l, more preferably 0.9 to 1.1 ml/l, and most preferably 1.0± 0.1 ml/l. The trace element solution is preferably used at 1x concentration. Typical ion concentrations in the medium will be: Fe, 200nM; Zn, 75nM; Co, 2nM; Mo, 2nM; Cu, 0.5nM; Mn, 50nM; B, 8.1nM; Se, 0.1nM. A preferred source of B is boric acid which, advantageously, inhibits precipitation.

The pH of the medium is preferably between about 6.5 and about 7.5, and is more preferably about 7.0. The pH of the medium can be increased by adding alkali, such as 3M NaOH.

Optionally, the medium may comprise 100 to 150µl/l antifoam, preferably 125 to 140 µl/l antifoam, and more preferably 133±0.02 µl/l antifoam. Any suitable non-biodegradable antifoam can be used in the present invention (*e.g*. polypropylene glycol *etc*.), provided that it inhibits the build-up of foam and/or reduces foam or trapped air by causing bubbles to burst (*e.g*. by reducing surface tension).

The medium may also comprise one or more supplements required for culturing auxotrophic cells, wherein the supplement provides the compound required by the cell. Supplements include vitamins and amino acids *e.g.* the medium may include thiamine (*e.g.* 5 to 20 mg/ml, preferably 8 to 12 mg/l, more preferably 9 to 11 mg/l, and most preferably 10±0.01 mg/l).

The medium may also comprise one or more antibiotics if the organism being grown is antibiotic-resistant. In selective media of this type, chloramphenicol is the preferred antibiotic, although other antibiotics may be used. Antibiotic levels of 10-30mg/ml are typical, preferably 18 to 22 mg/l, more preferably 19 to 21 mg/l, and most preferably 20±0.01 mg/l.

Although the medium preferably contains only defined components, in some situations it may be also include undefined organic nitrogenous compounds, although this is not preferred. Suitable organic nitrogenous compounds include casamino acids, FCS, BSA *etc.*

A defined component means a component that has a precise and completely defined composition. Defined components are generally chemically synthesised so that there is substantially no variation in the composition of the component. In contrast, an undefined component does not have a precise or completely defined composition. Generally, they include various undefined compounds and the composition of the component varies *e.g.* due to inherent biological variability.

Three specifically preferred embodiments of the cell culture medium have pH 7 and consist of:

| | | | |
|---|---|---|---|
| Glycerol (g/l) | 38±0.05 | 56.5±0.05 | 39.32±0.1 |
| (NH₄)₂SO₄ (g/l) | 7.26±0.012 | 7.26±0.012 | 7.26±0.01 |
| (NaPO₃)₇ (g/l) | 4.0±0.04 | 7.1±0.04 | 7.36±0.01 |
| MgSO₄.7H₂O (g/l) | 1.63±0.015 | 2.94±0.015 | 2.94±0.01 |
| K₂SO₄ (g/l) | 7.0±0.01 | 7.24±0.01 | 7.24±0.01 |
| trace element solution (ml/l) | 1.0±0.01 | 1.0±0.01 | 1.0±0.01 |

The medium is preferably sterile.

The medium of the invention can be manufactured using any standard technique. Preferably all the components of the medium are dissolved in de-ionised water and the resulting solution is filter-sterilised to give the medium of the invention. In a preferred embodiment, all the components of the medium, except K₂SO₄ and the optional antifoam, are dissolved in de-ionised water and filter sterilised. K₂SO₄ and the antifoam are separately dissolved in de-ionised water and autoclaved to sterilise. The sterile solutions are then combined to give the final medium.

The present invention also provides the use of the medium of the invention for culturing cells.

The medium can used to culture different cells, including prokaryotic cells (*e*.*g*. Gram +ve and -ve bacteria) and eukaryotic cells (*e.g*. yeasts, insect cells, mammalian cells *etc.).* Preferably the medium is used to cultivate prokaryotic cells such as *Escherichia coli, Helicobacter pylori, Haemophilus influenzae, Corynebacterium diphtheriae, Neisseria meningitidis, Bordetella pertussis* or *Clostridium tetani*. It is particularly useful for cultivating *E.coli.* Where eukaryotic cells are cultured, the pH of the medium may be in the region of 5.5, and additional supplements may be required

Preferably, the cells are cultured in the cell culture medium of the present invention for at least 6 hours, more preferably at least 36 hours, and most preferably at least 72 hours under suitable conditions for the production of the desired component.

The process can comprise any form of cell culture, including batch, fed batch or continuous culture *[e.g.* Moser (1985) Chapters 14-16 of *Fundamentals of Biochemical Engineering* (ed. Brauer)]. Preferably the process comprises batch culturing the cells.

The medium may be used to obtain a desired cell component. Preferred components are proteins (*e.g*. antigens, immunogenic proteins, enzymes, receptors, antibodies, *etc*.) and carbohydrates (*e.g*. structural carbohydrates, LPS *etc.*). It is particularly preferred that the desired component is an immunogenic component *i.e.* a component that is capable of stimulating the immune system of a human or animal. Such immunogenic components include those associated with the virulence of a bacteria.

The present invention also provides a process for producing a desired component comprising:
(1) culturing a cell capable of producing the desired component in the cell culture medium of the invention, under conditions leading to the production of the desired component; and
(2) isolating the desired component.

Preferred components include the *H.pylori* antigens VacA and CagA [*e*.*g*. see WO93/18150] and NAP [*e.g*. see WO96/01272, WO96/01273, and Evans *et al*. (1995)]. Other preferred components are described by Rappuoli *et al*. (1993).

Preferably, the desired component is *Helicobacter pylori* neutrophil activating protein (NAP), as the medium of the invention enables, surprisingly, the production of NAP at levels exceeding those obtained with a complex medium. NAP is constitutively expressed (i.e. its expression/production does not depend upon any induction mechanism), so it is important to optimise cultivation conditions in such a way that the carbon source is diverted to moderate cellular growth and to protein expression, rather than to produce inhibitory by-products. Inhibitory by-product formation can be avoided by using different feeding techniques for the carbon source, thus allowing better process control.

Another component which can be produced using the culture medium of the invention is ΔG287 protein [WO01/64922] derived from protein 287 [WO99/57280] from serogroup B *N.meningitidis*.

The immunogenic component may, where necessary, be genetically detoxified or treated by a toxoiding process. Methods for genetically detoxifying and toxoiding immunogenic components are well known to those skilled in the art and include those described by Rappuoli (1994).

The desired components may be present in the cell endogenously. Alternatively, the cell may be cultivated under conditions leading to the production of the desired component. The cell may be transformed with a nucleic acid molecule encoding the desired component, wherein the nucleic acid molecule comprises the necessary regulatory sequences for expression of the encoded component. Such regulator sequences may include not only a promoter, but additional regulatory sequences such as, in eukaryotes, an enhancer, splice sites and polyadenylation sequences. The regulatory sequences may be inducible or repressible leading to the control of expression of the nucleic acid molecule. For example, the promoter may be an inducible promoter such as the metalliothionine promoter or a heat shock promoter.

If the desired cell component is encoded by a gene expressed in a cell being cultured, it may be desirable to include an effector molecule in the medium that specifically regulates expression of the gene encoding the desired gene component. The effector may be an inducer which, on interaction with its corresponding responsive element, results in the induction of gene expression. Preferred inducers include IPTG, cyclic AMP, phorbol esters, heavy metals, glucocorticoid, progesterone, estrogen, thyroid hormone, retenoic acid *etc.*. It will be appreciated that the gene to be inducibly expressed will be operably linked to the relevant responsive element. The optimal time of induction can easily be determined empirically. The relationship between induction timing and the metabolic state of the host cell during the fermentation cycle may be used to optimise inducible protein production.

The desired component can be isolated from the cell culture using one or more standard techniques including those described by Manetti *et al*. (1995).

Suitable culture conditions for the production of the desired component, including the duration of the culture, will vary depending on the cell being cultured. However, one skilled in the art can easily determine the culture conditions required for the production of the desired component by following standard protocols, such as those described in the series *Methods in Microbiology,* Academic Press Inc. and, if necessary, by performing a number of standard experiments to determine suitable culture conditions.

The level of expression depends on the biochemical characteristics of the desired component, induction mechanisms of its gene expression, the host strain being used, and cultivation conditions such as temperature. It is therefore desirable to optimise the production of recombinant proteins. The optimisation process often involves improvement of the fermentation conditions focussing on medium composition (Rinas *et al*., 1989; Macdonald and Neway, 1990), growth conditions (Chalmers *et al*., 1990; Galindo, *et al*., 1990; Park and Ryu, 1990), induction method (Kopetzki *et al*., 1989; Okita *et al*., 1989), and minimum metabolite inhibition (Bauer *et al*., 1990; Konstantinov *et al*., 1991; Luli and Strohl, 1990) as well as its genetic systems. An important approach to optimise production of a desired protein during an cell fermentation has been to achieve maximum specific production using an appropriate culture condition (for constitutive protein expression) and an induction method (for inducible protein expression).

The components are preferably used to produce an immunogenic composition, such as a vaccine. This will typically involve combining the component with an adjuvant. Components useful for producing immunogenic compositions and vaccines include the *H.pylori* antigens listed above, and antigens for immunising against bacterial infections (*e.g*. type B gastritis, bacterial meningitidis, diphtheria, tetanus, whooping cough *etc.).*

The invention also provides a process for the production of a pharmaceutical composition (*e.g*. an immunogenic composition, such as a vaccine) comprising:
(1) culturing a cell capable of producing a therapeutic component in the culture medium of the invention under conditions leading to the production of the desired component;
(2) isolating the desired component; and
(3) combining the desired component with a suitable adjuvant.
The invention also provides a chemically-defined medium for use in expressing heterologous proteins in *E.coli*, wherein the medium contains (NaPO₃)₇ as the main phosphate source.

### BRIEF DESCRIPTION OF DRAWINGS

Figure 1 shows the results of producing NAP using a complex medium.
Figure 2 shows the results of producing NAP using chemically defined medium containing sodium polyphosphate.
Figure 3 shows the result of producing NAP using chemically defined medium containing glycerol as the carbon source (Figure 3a) and glucose as the carbon source (Figure 3b).
Figure 4 shows the result of producing NAP using chemically defined medium containing 1x trace element solution (Figure 4a) and 1000x trace element solution (Figure 4b).

### MODES FOR CARRYING OUT THE INVENTION

### Inoculum media

"Inoculum medium 1" was a complex medium containing (per litre of de-ionized water): Glycerol, 5.0 g; yeast extract (Difco), 5.0 g; yeast extract (PTK, Deutsche Hefewerke GmbH), 10.0 g; NaCl, 8.0 g; chloramphenicol, 20.0 mg. 30ml of this medium was prepared and pH was adjusted to 7.3 using 3M NaOH. 25ml of this medium was filter sterilised (0.2µm pore size, Millipore) in a sterile 300 ml conical flask.

"Inoculum medium 2" was a chemically defined medium (Lee & Lee, 1996) containing (per litre of de-ionized water): glycerol, 20 g; KH₂PO₄, 13.5 g; (NH₄)₂HPO₄, 4.0 g; MgSO₄.7H₂O, 1.4 g; citric acid, 1.7 g; thiamine, 10.0 mg; chloramphenicol, 20.0 mg. 30ml of this medium was prepared and pH was adjusted to 7.0 using 3M NaOH. 25ml of this medium was filter sterilised (0.2µm pore size, Millipore) in a sterile 300 ml conical flask.

### NAP production media

"NAP production medium 1" was a complex medium containing (per litre of de-ionized water): Glycerol, 5.0 g; yeast extract (Difco), 5.0 g; yeast extract (PTK, Deutsche Hefewerke GmbH), 10.0 g; NaCl, 8.0 g; chloramphenicol, 20.0 mg. The medium (1500 ml) was prepared according to Table 1:

| **Component number** | **Component name** | **Mass of component per 1500 ml** | **Method of preparation** **(all volumes are final)** |
|---|---|---|---|
| 1 | Glycerol | 7.5 g | Dissolved in 1200 ml of de-ionized water (in the reactor vessel, Applikon, Italia) |
| 2 | Yeast extract | 7.5 g | Dissolved in 95 ml of de-ionized water (in 250 ml glass bottle). |
| 3 | Yeast extract (PTK) | 15 g | Dissolved in 100 ml of de-ionized water (in 250 ml glass bottle). |
| 4 | NaCl | 12 g | Dissolved in 100 ml of de-ionized water (in 250 ml glass bottle). |

All components were autoclaved for 30 minutes at 121°C. Components 2, 3 and 4 were added to the reactor vessel (containing component i) aseptically inside a laminar flow chamber after the reactor vessel was cooled down to 40°C, as was chloramphenicol solution (1.5 ml of 20.0 mg.ml⁻¹ stock, filter sterilised). The pH of the medium was adjusted in the reactor to 7.3 by automatic addition of 3M NaOH. The 1500ml final volume of the volume consists of 1495ml components 1-4, and 5ml of chloramphenicol stock solution and added NaOH.

"NAP production medium 2" was a chemically defined medium, prepared from "basal medium" and "1X trace element solution (1ml/l)".

The "basal medium" contained (per litre of de-ionized water): Glycerol, 38 g; (NE₄)₂SO₄, 7.26 g; sodium polyphosphate (NaPO₃)₇ (Budenheim GmbH), 4.0 g; MgSO₄.7H₂O, 1.63 g; K₂SO₄, 7.0 g; thiamine, 10.0 mg; chloramphenicol, 20.0 mg; antifoam (Polypropylene glycol, Aldrich), 133.0 µl. The medium (1500 ml) was prepared according to **Table 2:**

| **Component number** | **Component name** | **Mass of component per 1500 ml** | **Method of preparation (all volumes are final)** |
|---|---|---|---|
| 1 | Glycerol | 57 g | Dissolved in 500 ml of de-ionised water and filter sterilised using vacuum manifold. |
| 2 | (NH₄)₂SO₄ | 3.75 g | |
| 3 | (NaPO₃)₇ | 6.0 g | |
| 4 | MgSO₄.7H₂O | 2.52 g | |
| 5 | K₂SO₄ | 10.5 g | Dissolved in 990 ml of de-ionised water (in reactor vessel). |

Component 5 was autoclaved for 30 minutes at 121°C. A mixture of components 1, 2, 3, and 4 together with thiamine solution (1.5 ml of 10 mg/ml stock, filter sterilised), chloramphenicol solution (1.5 ml of 20 mg/ml stock, filter sterilised), and antifoam (autoclaved separately in glass bottle) was added to the reactor vessel aseptically inside a laminar flow chamber after the reactor vessel was cooled down to 40°C. The pH of the medium was adjusted in the reactor to 7.0 by automatic addition of 3M NaOH. The 1500ml final volume of the volume consists of 1490ml components 1-4, and 10ml of chloramphenicol and thiamine stock solutions, antifoam, inoculum, and added NaOH.

The "trace element solution" was prepared at 1000x strength by dissolving the following inorganic compounds in a final volume of 1000ml de-ionized water: H₂SO₄ (37%), 10 ml; FeCl₃.6H₂O, 54.0g; ZnSO₄.7H₂O, 22.0g; CoCl₂.6H₂O, 0.5g; Na₂MoO₄.2H₂O, 0.5g; CuSO₄.5H₂O, 0.13g; boric acid, 0.5g; MnSO₄.H₂O, 11.0g; sodium selenite, 0.02g. The solution was filter sterilised in a sterile bottle and was stored at +4°C. For use, a 1x dilution was prepared by diluting 1000X solution with sterile de-ionized water.

### Preparation of inoculum

The *H.pylori* NAP gene was amplified by PCR and inserted into the *E.coli*/*B.subtilis* shuttle vector pSM214 (ATCC 67320). Chloramphenicol resistance was used as the selection marker. The plasmid was used for the transformation of *E.coli* MM294.1 (ATCC 33625), which is an auxotroph for thiamine. The recombinant organism was named *"E.coli* MM 294.1 NAPB5 S784P8WS1" and was stored as 300µl aliquots of culture in 10% glycerol (v/v) at -70°C.

250µl of frozen stock of *E.coli* was inoculated into 25ml of the inoculum media described above in sterile 300ml conical flasks. The flasks were incubated in temperature-controlled rotary shakers for 8 h at 35°C and at 200 rpm (inoculum 1) or for 14 h at 35°C and at 150 rpm (inoculum 2). 2ml (inoculum 1) or 4.5ml (inoculum 2) of this culture was inoculated to 1500ml of NAP production medium 1 or 2, respectively.

### Batch bioreactor

NAP production was carried out in a 3.0 litre batch bioreactor (Applikon Italia; Pyrex glass vessel; diameter (dₜ) = 13.0 cm; height (h) = 23.4 cm, h/dₜ = 1.8; this is the same reactor as used above during medium preparation) with a working volume of 1.5 1. The bioreactor was equipped with two six-bladed disc turbine impellers (impeller diameter (dᵢ) = 4.5 cm, dᵢ/dₜ = 0.35, direct stirring by top mounted motor), air sparger, pH probe (combined electrode, AppliSens) and DO probe (Mettler Toledo). A digital control unit (ADI1030) connected to a microcomputer loaded with BioXpert process control software (Version 1.14, Applikon Italia) was used to control pH, dissolved oxygen tension, agitation and temperature, and also to collect on-line data. Agitation was kept constant at 650 rpm. The dissolved oxygen level was controlled automatically at 75% of saturation by sparging air or a mixture of air and pure oxygen when necessary. Temperature was maintained at 35°C with a thermostatic water circulator. pH of the culture medium was maintained at 7.3 (complex medium) and at 7.0 (chemically defined medium) using 3M NaOH. Foam was suppressed, when necessary, by the addition of antifoam agent (polypropylene glycol). The cultivation in the reactor was carried out for at least 24 hours. Samples were withdrawn at regular interval and the growth was checked immediately by measuring OD₅₉₀ₙₘ. Samples were then stored in sterile tubes at -20°C for further analysis.

### Analytical methods

To estimate cell mass, cellular growth was monitored by measuring OD₅₉₀ₙₘ with a UV/VIS spectrophotometer (Novaspec II, Pharmacia-KKB). 1ml sample was then dispensed in a pre-weighed Eppendorf tube and was centrifuged at 13000rpm for 10 minutes. The supernatant was stored for further analysis of residual glycerol or glucose. The pellet was washed with de-ionized water and then dried at 37°C till a constant weight was obtained. The cell mass was expressed in terms of cell dry weight (CDW, g/l).

To measure NAP levels in samples, 1ml of diluted cell sample was washed with PBS and centrifuged. The pellets were re-suspended in reducing sample buffer containing DTT. Suspensions were incubated in a boiling water bath for 10 minutes. Samples were then subjected to SDS-PAGE (12.4% polyacrylamide) followed by staining with Coomassie brilliant blue R250. Quantification of NAP was carried out by densitometry (Image Master 1D Elite, version 3.0, Pharmacia), with a calibration curve based on different known concentrations of purified NAP. Amount of NAP was expressed in terms of mg/l.

Residual glycerol levels were estimated using an analytical kit (Boehringer Mannheim). Residual glucose was estimated by an automatic enzymatic glucose analyser (Yellow Springs Instruments Co).

### NAP production results

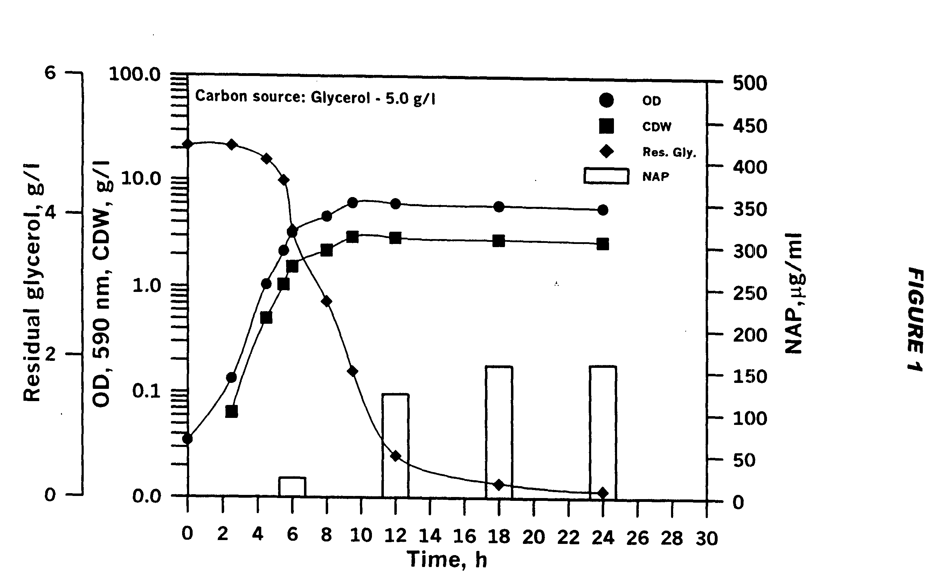

Figure 1 shows the cultivation profile in complex medium. The organism reached stationary phase at the tenth hour, as indicated by optical density data and supported by CDW data. The organism was allowed to be in stationary phase for an additional 14 hours. The production profile of NAP reveals that the expression of this protein takes place mainly in the stationary phase. The production levels of NAP at the 18th and 24th hour were 157.53 mg/l and 159.16 mg/l respectively. The corresponding CDWs were 2.79 g/l in both cases. As NAP is an intracellular protein, production was expressed in terms of specific production at the 24th hour of cultivation ― at this stage, the specific production was 57.05 mg/g CDW.

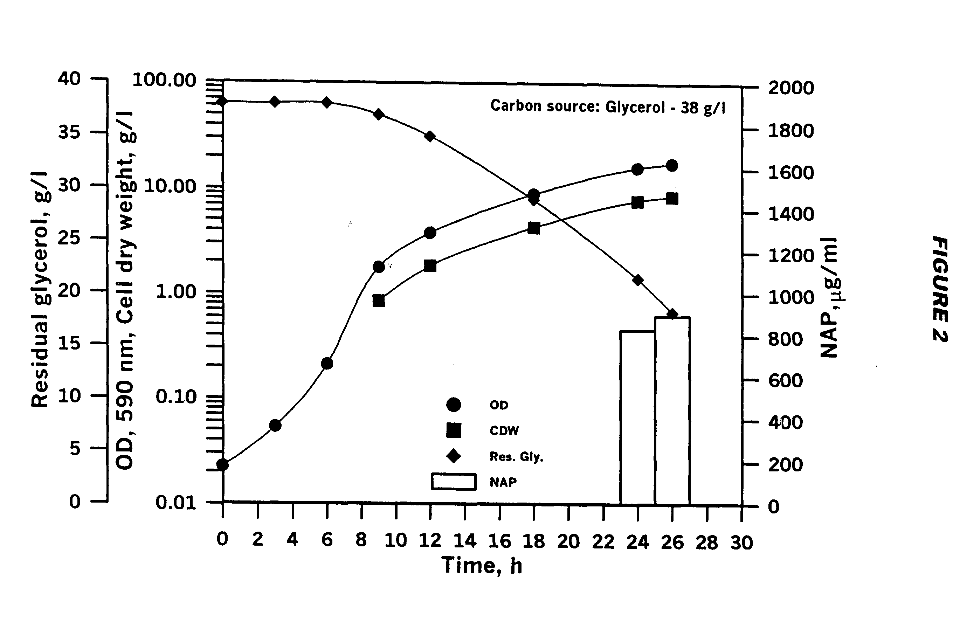

Figure 2 shows the cultivation profile in defined medium. The organism reached stationary phase at the 24th hour of cultivation. However, it is evident from optical density and CDW data that the organism continued to grow after the 24th hour of cultivation, although the difference in growth rate was not substantial. The growth occurred because of the presence of carbon source in the medium, which was not consumed completely at the 26th hour of cultivation. The cultivation however, was terminated at the 26th hour. The production levels of NAP at the 24th hour and 26th hour were 829.71 mg/l and 897.19 mg/l, respectively. The corresponding specific production was 106.10 mg/g and 104.20 mg/g.

Advantageously, therefore, the chemically-defined medium results in increased levels of NAP production and NAP specific production. After 24 hours, the increase was about 5.21 fold (production) and 1.85 fold (specific production) over that obtained using complex medium:

| **Medium** | **OD**_{**590**} | **CDW** **(g/l)** | **NAP** **(mg/l)** | **Specific NAP** **(mg/g)** |
|---|---|---|---|---|
| Complex | 5.8 | 2.79 | 159.16 | 57.05 |
| Defined | 16.3 | 7.82 | 829.71 | 106.10 |

Thus the medium of the invention avoids the problems associated with using complex media, provides the benefits associated with chemically-defined media (*e.g.* lot-to-iot consistency) and, in addition, beneficially leads to increased levels of production of NAP.

### Trace element concentration

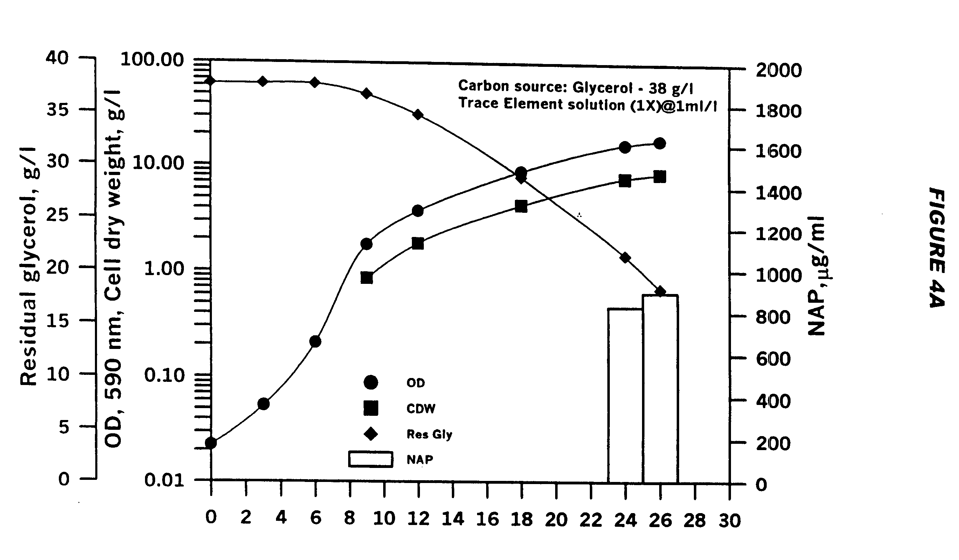

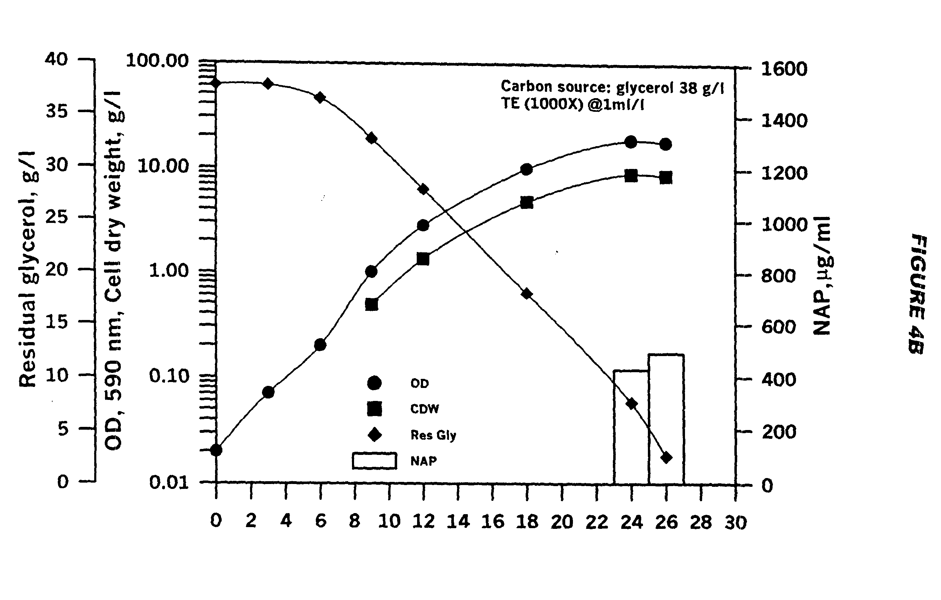

Figure 4 shows results of experiments performed using both 1000X and 1X trace element solution in the medium. While the specific growth rate of recombinant bacteria in these two media are almost same (0.44 h⁻¹ and 0.46 h⁻¹ for 1000X and 1X, respectively), the amount of glycerol consumed at the 24th hour (93.42% and 51.82% of the initial glycerol for 1000X and 1X, respectively) and the amount of NaOH consumed for the maintenance of the pH at 7.0 at the same time (95ml and 60ml for 1000X and 1X, respectively) were higher when using 1000X trace element solution. The results clearly show that concentrated trace element solution encouraged conversion of carbon source to organic acid, which is a wasteful by-product. The NAP data also support this ― production levels at 24th hour were 429.75 mg/l and 829.71 mg/l for the medium containing 1000X and 1X trace element solution, respectively. Specific production of NAP was 47.67 mg/g and 106.10 mg/g for 1000x and 1X, respectively. Thus NAP production level was higher by 193% and the specific production of NAP was higher by 223% when 1X trace element solution was used instead of 1000x.

### Glucose as a carbon source

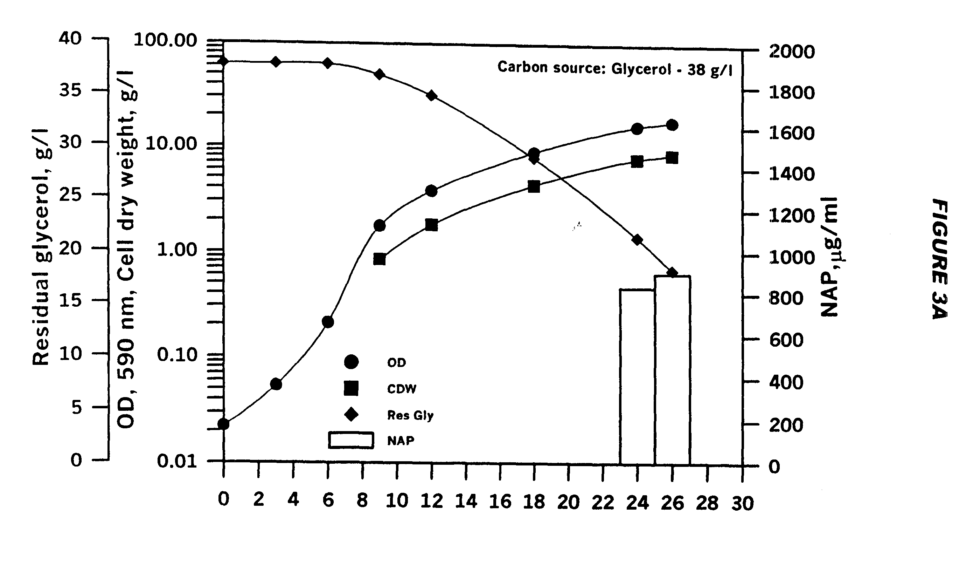

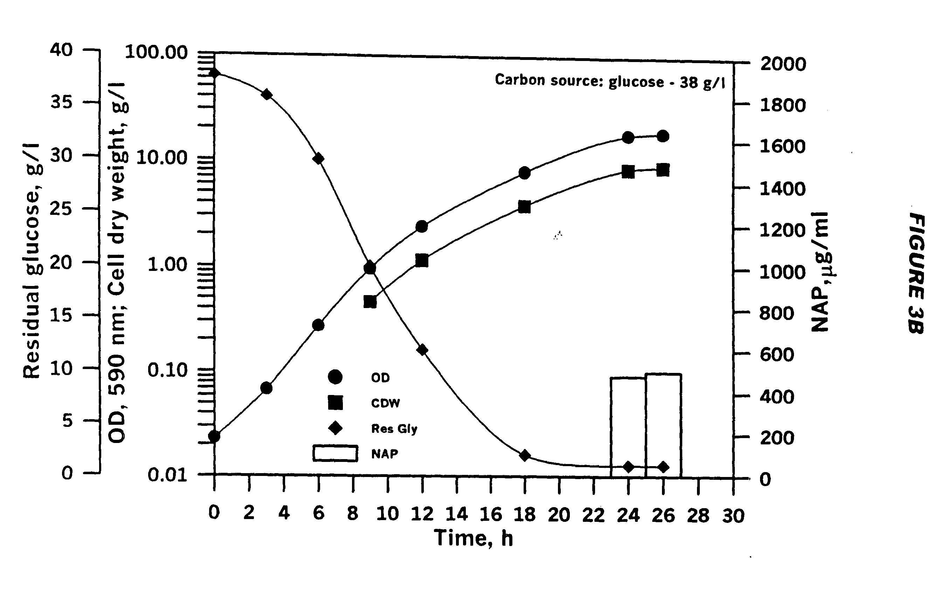

Glucose was substituted at the same concentration for glycerol, with the results shown in Figure 3. Whilst depletion of glucose at 24 hours was almost complete, cellular growth was only 5.79% higher than obtained using glycerol (53% depletion). The amount of alkali consumed over 24 hours to maintain pH was also higher when glucose was the carbon source (90ml *vs*. 60ml). This suggests that the use of glucose favours the formation of organic acids.

NAP production after 24 hours was 829.71 mg/l (glycerol) and 478.99 mg/l (glucose), with specific production levels being 106.10 mg/g (glycerol) and 86.06 mg/g (glucose). Glycerol is therefore a better carbon source than glucose for NAP production.

### Medium optimisation

To improve the medium, the central composite rotatory design (CCRD) method of Box & Wilson was used.

Three input variables (X₁, X₂, X₃) were used (carbon:nitrogen ratio, sodium polyphosphate concentration, MgSO₄.7H₂O concentration).These were varied as follows:

| **Variable** | **X**_{**n**} | **Level** | | | | |
|---|---|---|---|---|---|---|
| | | **-1.68** | **-1** | **0** | **+1** | **+1.68** |
| C:N | X₁ | 1.6 | 5.0 | 10.0 | 15.0 | 18.4 |
| Polyphosphate | X₂ | 0.64 | 2.0 | 4.0 | 6.0 | 7.36 |
| MgSO₄.7H₂O | X₃ | 0.26 | 0.8 | 1.6 | 2.4 | 2.94 |

The output variable was NAP production (µg/ml).

The following results were obtained experimentally:

| **Experiment** | **X**_{**1**} | **X**_{**2**} | **X**_{**3**} | **NAP** |
|---|---|---|---|---|
| 1 | -1 | -1 | -1 | 833.71 |
| 2 | +1 | -1 | -1 | 352.35 |
| 3 | -1 | +1 | -1 | 544.12 |
| 4 | +1 | +1 | -1 | 941.89 |
| 5 | -1 | -1 | +1 | 556.52 |
| 6 | +1 | -1 | +1 | 827.57 |
| 7 | -1 | +1 | +1 | 730.93 |
| 8 | +1 | +1 | +1 | 812.04 |
| 9 | 0 | 0 | 0 | 963.33 |
| 10 | 0 | 0 | 0 | 930.41 |
| 11 | -1.68 | 0 | 0 | 130.59 |
| 12 | +1.68 | 0 | 0 | 664.96 |
| 13 | 0 | -1.68 | 0 | 379.64 |
| 14 | 0 | +1.68 | 0 | 1107.71 |
| 15 | 0 | 0 | -1.68 | 910.47 |
| 16 | 0 | 0 | +1.68 | 918.13 |
| 17 | 0 | 0 | 0 | 855.68 |

These results were fitted to a quadratic function with ten parameters. This produced the following optimised values for the medium:

| **Input variable** | **Optimised value** |
|---|---|
| Carbon:Nitrogen ratio | 14.4 |
| Sodium polyphosphate | 7.1 g/l |
| Magnesium sulphate | 2.94 g/l |

With these optimised values, NAP production of 1132.2µg/ml was predicted.

The following media were used experimentally:

| **Component** | **Un-optimsed** | **Optimsed** |
|---|---|---|
| Glycerol | 38.0 g/l | 56.61 g/l |
| (NH₄)₂SO₄ | 7.26 g/l | |
| (NaPO₄)₇ | 4.0 g/l | 7.1 g/l |
| MgSO₄.7H₂O | 1.6 g/l | 2.94 g/l |
| K₂SO₄ | 7.24 g/l | |
| Trace element (1x) | 1.0 ml/l | |
| Thiamine | 10 mg/ml | |
| Chloramphenicol | 20 mg/ml | |
| Antifoam | 500 µl/l | |

Expression levels of NAP were as follows

| | | |
|---|---|---|
| NAP production | 829.71 µg/ml | I 1184.6 µg/ml |

Production in optimised medium was within 1% of the predicted level and was 43% higher with the previous (un-optimsed) experiments.

### Expression of ΔG287

The ΔG287 derivative of serogroup B *N.meningitidis* protein 287 was expressed in *E.coli* BL21. Cells were cultured at 35±1°C under 650 rpm agitation at 30% dissolved oxygen in 1.5 litre stirred tank reactors. The culture medium was optimised as follows:

| **Component** | **Un-optimsed** | **Optimsed** |
|---|---|---|
| Glycerol | 39.32 g/l | 72.34 g/l |
| (NH₄)₂SO₄ | 7.26 g/l | |
| (NaPO₄)₇ | 4.0 g/l | 7.36 g/l |
| MgSO₄.7H₂O | 1.6 g/l | 2.94 g/l |
| K₂SO₄ | 7.24 g/l | |
| Trace element (1x) | 1.0 ml/l | |
| Thiamine | 10 mg/ml | |
| Kanamycin | 20 mg/ml | |
| Antifoam | 500 µl/l | |
| pH | 7.0±0.1 | |

Expression levels of ΔG287 were as follows

| | | |
|---|---|---|
| ΔG₂87 production | 325.74 µg/ml | 670.45 µg/ml |

Optimisation of the medium therefore doubled expression levels of ΔG287.

It will be understood that the invention has been described by way of example only and modifications may be made whilst remaining within the scope and spirit of the invention. Other embodiments will be apparent to those skilled in the art.

### REFERENCES

Bauer *et al*. (1990) *Appl. Environ. Microbiol.* 56: 1296-1302
Bonsignore *et al*. (1989) *Abstr. Pap. Am. Chem. Soc.* 198 Meet, MBTD 199
Box & Wilson (1951) *J. Roy. Statist. Soc.* B13:1-45
Chalmers *et al*. (1990) *Appl. Environ. Microbiol.* 56: 104-111
Comberbach *et al*. (1991) EP-A2-0414374
Evans *et al*. (1995) *Gene* 153:123-127
De Vuyst, L. (1995) *J. Appl. Bacteriol.* 78: 28-33
Galindo *et al*. (1990) *J. Ferm. Bioeng.* 69: 159-165
Konstantinov *et al*. (1991) *J. Ferm. Bioeng.* 71: 350-355
Kopetzki *et al*. (1989) *Mol. Gen. Genet.* 216: 149-155
Lee & Lee (1996) *Journal of Environmental Polymer Degradation* 4(2):131-134
Luli & Strohl (1990) *Appl. Environ. Microbiol.* 56: 640-645
MacDonald & Neway (1990) *Appl. Environ. Microbiol.* 56: 640-645.
Manetti *et al*. (1995) *Infect. Immun.* 63:4476-4480.
Okita *et al*. (1989) *Biotechnol. Bioeng.* 34: 854-862
Park & Ryu (1990) *Biotechnol. Bioeng.* 35: 287-295
*Rappuoli et al*. (1993) *European Journal of Gastroenterology and Hepatology of Helicobacter pylori infection,* Proceedings of an interdisciplinary meeting (Genova, June 18-19, 1993) J.J. Misiewicz, Ed. (CS Current Science), pp S76-S78.
Rappuoli (1994) *Vaccine* (1994) 12:579-581.
Rinas *et al*. (1989) *Appl. Microbiol. Biotechnol.* 31: 163-167
Stephenne, J. (1990) *Vaccine* 8: S69-S73
Stratakalaitis *et al*. (1991) *Abstr. Gen. Meet. Am. Soc. Microbiol.* 91 Meet, 190
Zhang *et al*. (1998) *in:* Kelley & Ramelmeier (eds) *ACS Symposium Ser* 698: 12-27
Zhang & Greasham (1999) *Appl. Microbiol. Biotechnol.* 51: 407-421

## Claims

1. A cell culture medium comprising glycerol or glucose, an ammonium salt, sodium polyphosphate, a magnesium salt, a potassium salt and trace elements, and having a pH between 5.5 and 8.0.

2. The medium of claim 1, comprising glycerol, (NH₄)₂SO₄, (NaPO₃)₇, MgSO₄.7H₂O, K₂SO₄, Fe, Zn, Co, Mo, Cu, Mn, B and Se.

3. The medium of any preceding claim, comprising:
glycerol or glucose: 6.0 to 150.0 g/l;
(NH₄)₂SO₄ : 2.0 to 9.0 g/l;
(NaPO₃)₇ or (NaPO₃)₆: 0.6 to 12.0 g/l;
MgSO₄.7H₂O : 0.1 to 6.0 g/l; and
K₂SO₄ : 0.1 to 8.0 g/l.

4. The medium of any preceding claim, having a carbon/nitrogen ratio between 4 and 20.

5. The medium of any preceding claim, further comprising antifoam.

6. The medium of any preceding claim, further comprising a supplement required for culturing auxotrophic cells.

7. The medium of any preceding claim, further comprising one or more antibiotics.

8. The medium of any preceding claim, wherein the medium is sterile.

9. The medium of any preceding claim, having pH 7 and comprising:
38±0.05 g/l glycerol;
7.26±0.012 g/l (NH₄)₂SO₄;
4.0±0.04 g/l (NaPO₃)₇;
1.63±0.015 g/l MgSO₄.7H₂O; and
7.0±0.01 g/l K₂SO₄;

10. The medium of any preceding claim, having pH 7 and comprising:
56.5±0.05 g/l glycerol;
7.26±0.012 g/l (NH₄)₂SO₄;
7.1±0.07 g/l (NaPO₃)₇;
2.94±0.03 g/l MgSO₄.7H₂O; and
7.24±0.01 g/l K₂SO₄;

11. The medium of any preceding claim, having pH 7 and comprising:
72.34±0.05 g/l glycerol;
7.26±0.012 g/l (NH₄)₂SO₄;
7.36±0.07 g/l (NaPO₃)₇;
2.94±0.03 g/l MgSO₄.7H₂O; and
7.24±0.01 g/l K₂SO₄;

12. A process for culturing a cell, using the medium of any preceding claim.

13. A process for culturing bacteria, using the medium of any preceding claim.

14. The process of claim 13, wherein the bacteria is *E.coli*.

15. The process of any one of claims 12 to 14, wherein cells are cultured for at least 6 hours

16. A process for producing a component from a cell, comprising:
(1) culturing a cell capable of producing the component in the medium of any one of claims 1 to 11, under conditions leading to the production of the desired component; and
(2) isolating the component.

17. The process of claim 16, wherein the component is a protein.

18. The process of claim 17, wherein the protein is a bacterial antigen.

19. The process of claim 18, wherein the antigen is a *H.pylori* antigen.

20. The process of claim 19, wherein the *H.pylori* antigen is NAP and the cell is *E.coli.*

21. The process of claim 17, wherein the component is ΔG287.

22. A process for the production of a pharmaceutical composition, comprising:
(1) culturing a cell capable of producing the component in the medium of any one of claims 1 to 11, under conditions leading to the production of the desired component,
(2) isolating the component, and
(3) combining the desired component with a suitable adjuvant.
